(19) Europäisches Patentamt
European Patent Office
Office européen des brevets

(11) Veröffentlichungsnummer: **0 272 210 A2**

(12) # EUROPÄISCHE PATENTANMELDUNG

(21) Anmeldenummer: **87810712.7**

(22) Anmeldetag: **02.12.87**

(51) Int. Cl.4: **C07D 209/76**, C08G 73/12, C09J 3/00

(30) Priorität: **08.12.86 CH 4887/86**

(43) Veröffentlichungstag der Anmeldung: **22.06.88 Patentblatt 88/25**

(84) Benannte Vertragsstaaten: **CH DE FR GB IT LI NL**

(71) Anmelder: **CIBA-GEIGY AG**
**Klybeckstrasse 141**
**CH-4002 Basel(CH)**

(72) Erfinder: **Renner, Alfred, Dr.**
**Marcoup 2**
**CH-3286 Muntelier(CH)**

(54) **(Poly)oxaalkylen-alpha, omega-Bisimide der Allyl-bicyclo[2.2.1]hept-5-en-2,3-dicarbonsäure und deren Verwendung.**

(57) Imide der Formel I

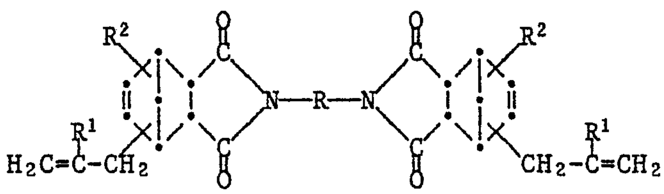

(I),

worin $R^1$ und $R^2$ unabhängig voneinander ein Wasserstoffatom oder Methyl bedeuten, R einen (Poly)oxa-alkylenrest der Formel

$$-[(C_pH_{2p}O)_s(C_qH_{2q}O)_tC_rH_{2r}]-$$

darstellt mit p, q und r = unabhängig voneinander eine ganze Zahl von 2 bis 6, s=0 oder 1 und t=1-30, eignen sich zur Herstellung von vernetzten Polymeren, insbesondere als Zähigkeitsverbesserer in Stoffgemischen mit anderen ungesättigten Imiden.

EP 0 272 210 A2

## (Poly)oxaalkylen-α,ω-Bisimide der Allyl-bicyclo[2.2.1]hept-5-en-2,3-dicarbonsäure und deren Verwendung

Die Erfindung betrifft neue (Poly)oxa-alkylen-α,ω-Bisimide der Allyl-bicyclo[2.2.1]hept-5-en-2,3-dicarbonsäure, ein Verfahren zu deren Herstellung und deren Verwendung, gegebenenfalls zusammen mit anderen ungesättigten Imiden, zur Herstellung von Polymeren durch Erwärmen.

In den europäischen Patentveröffentlichungen 0 105 024 und 0 152 372 sind allyl-oder methallylsubstituierte (Methyl)Bicyclo[2.2.1]hept-5-en-2,3-dicarbonsäureimide beschrieben, aus denen durch Erhitzen vernetzte Polymere hergestellt werden können. Die europäische Patentveröffentlichung 0 166 693 beschreibt hydroxylgruppenhaltige bicyclische Imide und deren Verwendung als Härter für Epoxidharze.

Gegenstand der Erfindung sind Imide der Formel I

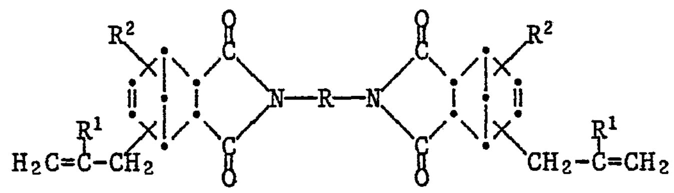

(I),

worin $R^1$ und $R^2$unabhängig voneinander ein Wasserstoffatom oder Methyl bedeuten, R einen (Poly)oxa-alkylenrest der Formel

$$-\!\!\left[(C_pH_{2p}O)_s(C_qH_{2q}O)_t C_rH_{2r}\right]\!\!-$$

darstellt mit p, q und r = unabhängig voneinander eine ganze Zahl von 2 bis 6, s = 0 oder 1 und t = 1-30.

Vorzugsweise bedeuten $R^1$und $R^2$ ein Wasserstoffatom.

Bevorzugt sind Imide der Formel I, worin s = 1, die Indizes p und r gleich sind und t = 1-20, und besonders bevorzugt solche, worin p und r jeweils 2 oder insbesondere 3 sind, q 2 bis 4 ist und t = 1-10.

Bevorzugt sind auch Verbindungen der Formel I, worin s = 0, die Indizes q und r gleich sind und t = 1-20, und besonders bevorzugt solche, worin q und r jeweils 2, 3 oder 4 sind und t = 1-10.

Die Alkylengruppen im (Poly)oxa-alkylenrest R der Imide der Formel I können unabhängig voneinander geradkettig oder verzweigt sein, wobei geradkettige Alkylengruppen bevorzugt sind. Geeignete Alkylengruppen sind z.B. Ethylen, 1,2-und 1,3-Propylen, 1,2-, 1,3-und 1,4-Butylen, Pentylen oder Hexylen.

Bevorzugte (Poly)oxa-alkylenreste R der Imide der Formel I sind, bei s = 1, $-(CH_2)_3OCH_2CH_2O(CH_2)_3-$, $-(CH_2)_3OCH_2CH_2CH_2CH_2O(CH_2)_3-$, $-(CH_2)_3O(CH_2CH_2O)_2(CH_2)_3$-oder $-(CH_2)_3O(CH_2CH_2CH_2CH_2O)_9(CH_2)_3-$, und bei s = 0 $-(CH_2CH_2O)_tCH_2CH_2-$, $-(CH_2CH_2CH_2O)_tCH_2CH_2CH_2-$, $-(CH_2\underset{|}{\overset{CH_3}{C}}HO)_tCH_2\underset{|}{\overset{CH_3}{C}}H-$ oder $-(CH_2CH_2CH_2CH_2O)_tCH_2CH_2CH_2CH_2-$, insbesondere solche mit t = 1-10. Ganz besonders bevorzugt sind Imide der Formel I, worin $R^1$ und $R_2$ Wasserstoff sind und R $-(CH_2)_3OCH_2CH_2O(CH_2)_3-$, $-(CH_2)_3O(CH_2CH_2CH_2CH_2O)_9(CH_2)_3$-und insbesondere $-(CH_2)_3O(CH_2CH_2O)_2(CH_2)_3$-oder $-(CH_2)_3OCH_2CH_2CH_2CH_2O(CH_2)_3$-bedeutet.

Die Imide der Formel I können auf an sich bekannte Weise hergestellt werden, indem man ein Anhydrid der Formel II

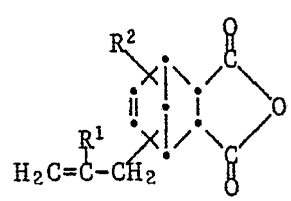

(II)

bei erhöhter Temperatur und unter Abdestillieren des bei der Reaktion entstehenden Wassers mit einem Diamin der Formel III

$H_2N-R-NH_2$ (III)

umsetzt, wobei für $R^1$, $R_2$ und R das oben Angegebene gilt.

Die Umsetzung kann ohne Lösungsmittel oder in Gegenwart eines inerten Lösungsmittels, das für die azeotrope Entfernung des Wassers verwendbar ist (Schleppmittel, wie Toluol und Xylole) erfolgen. Die Temperaturen für die Umsetzung in Gegenward eines Lösungsmittels können zwischen 100°C und Rückflusstemperatur liegen. Die Umsetzung in der Schmelze erfolgt zweckmässig bei Atmosphärendruck und/oder im Vakuum bei Temperaturen zwischen 100 und 250°C, insbesondere 130 und 220°C. Die Umsetzung in der Schmelze ist bevorzugt.

Die Verbindungen der Formeln II und III sind bekannt oder können nach an sich bekannten Methoden hergestellt werden. Anhydride der Formel II können z.B. nach dem in der US-PS 3,105,839 beschriebenen Verfahren erhalten werden, indem man Natrium-Cyclopentadienid oder Natrium-Methylcyclopentadienid mit einem Allyl-oder Methallylhalogenid umsetzt, worauf sich eine Diels-Alder-Reaktion mit Maleinsäureanhydrid anschliesst. Obgleich in der US Patentschrift angegeben wird, dass die Allylgruppe in 7-Stellung des bicyclischen Systems gebunden ist, zeigen neuere Untersuchungen, dass eine isomere Mischung in Bezug auf die Stellung der Allyl-bzw. Methallylgruppe (in 1-und 6-Position) und auch auf die Endo-und Exokonfiguration des Anhydridteils gebildet wird.

Diamine der Formel III können z.B. durch Cyanethylierung von (Poly)etherdiolen in Gegenwart eines basischen Katalysators und anschliessende Hydrierung der Dicyanoverbindung hergestellt werden. Die Cyanethylierung ist z.B. in Organic Reactions 5, 79-135 (1949) beschrieben. Einige Diamine der Formel III sind als Handelsprodukte der Firma BASF erhältlich.

Die Imide der Formel I fallen im allgemeinen in Form fester oder sehr zähflüssiger Harze an, die zu vernetzten Produkten mit vorzüglichen Eigenschaften polymerisiert werden können. Sie können vielseitig angewendet werden, z.B. als hitzebeständige Klebstoffe, als Isoliermaterialien in der Elektronik und Elektrotechnik und vor allem als Matrixharze für Verbundwerkstoffe. Die Imide der Formel I können allein oder zusammen mit anderen ungesättigten Imiden polymerisiert werden. Die Polymerisation wird zweckmässig bei Temperaturen zwischen 180 und 300°C, vorzugsweise 200-260°C, durchgeführt. Die Reaktionszeiten liegen im allgemeinen zwischen 1 und 50, besonders 2 und 12 Stunden. In manchen Fällen kann es auch vorteilhaft sein, die Polymerisation in zwei Stufen vorzunehmen, indem man zuerst bei relativ niedrigen Temperaturen, z.B. zwischen 180 und 220°C, eine Vorgelierung durchführt und anschliessend bei erhöhten Temperaturen, z.B. zwischen 240 und 300°C, auspolymerisiert.

Gegenstand der Erfindung sind daher auch Polymere, die dadurch erhältlich sind, dass man mindestens ein Imid der Formel I auf Temperaturen zwischen 180 und 300°C erhitzt.

Eine bevorzugte Verwendung der erfindungsgemässen Imide ist in Gemischen mit anderen ungesättigten Imiden, wobei die gehärteten Polymere sowohl eine hohe Glasumwandlungstemperatur und Hitzebeständigkeit als auch ausgezeichnete Zähigkeitseigenschaften aufweisen.

Härtbare Kunststoffe mit hoher Glasumwandlungstemperatur sind im allgemeinen spröd. Ueberraschenderweise sind die durch Härtung der unten beschriebenen erfindungsgemässen Stoffgemische erhaltenen Polymere viel zäher als Polymere aus den einzelnen Komponenten. Dabei wirken die erfindungsgemässen Imide der Formel I als Zähigkeitsverbesserer (Toughening Agent).

Gegenstand der Erfindung sind somit auch heisshärtbare Stoffgemische enthaltend

(a) 10-90 Gew.% mindestens eines Imids der Formel I nach Anspruch 1,

(b) 90-10 Gew.% mindestens eines ungesättigten Imids verschieden von (a) und, bezogen auf die Summe von (a) und (b),

(c) 0-30 Gew.% mindestens einer zur Reaktion mit der Komponente (a) und/oder (b) befähigten Verbindung.

Als ungesättigte Imide kommen als Komponente (b) z.B. solche der in den europäischen Patentveröffentlichungen 0 105 024 und 0 152 372 beschriebenen Art, oder Polymaleinimide, wie Z.B. in den US Patentschriften 3,562,223, 3,658,764, 3,380,964 und 4,038,251 oder in der DE-OS 23 50 472 beschrieben, in Betracht.

Beispiele für spezifische Imide als Komponente (b) sind:
Allyl-bicyclo[2.2.1]hept-5-en-2,3-dicarbonsäure-N-methylimid,
Allyl-bicyclo[2.2.1]hept-5-en-2,3-dicarbonsäure-N-allylimid,
Allyl-bicyclo[2.2.1]hept-5-en-2,3-dicarbonsäure-N-(2-ethylhexyl)imid,
Allyl-bicyclo[2.2.1]hept-5-en-2,3-dicarbonsäure-N-cyclohexylimid,
Allyl-bicyclo[2.2.1]hept-5-en-2,3-dicarbonsäure-N-phenylimid,
Allyl-bicyclo[2.2.1]hept-5-en-2,3-dicarbonsäure-N-benzylimid,
N,N'-Ethylen-bis-(allyl-bicyclo[2.2.1]hept-5-en-2,3-dicarbonsäureimid),
N,N'Hexamethylen-bis-(allyl-bicyclo[2.2.1]hept-5-en,2,3-dicarbonsäureimid),
N,N'-Dodecamethylen-bis-(allyl-bicyclo[2.2.1]hept-5-en-2,3-dicarbonsäureimid),
Bis-[4-(allyl-bicyclo[2.2.1]hept-5-en-2,3-dicarbonsäureimidophenyl)]methan,

Bis[4-(methallyl-bicyclo[2.2.1]hept-5-en-2,3-dicarbonsäureimidophenyl)]methan,
N,N'-p-Phenylen-bis(allyl-bicyclo[2.2.1]hept-5-en-2,3,-dicarbonsäureimid,
Bis[4-(allyl-bicyclo[2.2.1]hept-5-en-2,3-dicarbonsäureimidophenyl)]ether,
Bis[4-(allyl-bicyclo[2.2.1]hept-5-en-2,3-dicarbonsäureimidophenyl)]sulfon,
Allyl-methyl-bicyclo[2.2.1]hept-5-en-2,3-dicarbonsäure-N-allylimid,
Allyl-methyl-bicyclo[2.2.1]hept-5-en-2,3-dicarbonsäure-N-(2-ethylhexyl)imid,
Allyl-methyl-bicyclo[2.2.1]hept-5-en-2,3-dicarbonsäure-N-phenylimid,
N,N'-Hexamethylen-bis-(allyl-methyl-bicyclo[2.2.1]hept-5-en-2,3-dicarbonsäureimid),
Bis[4-(allyl-methyl-bicyclo[2.2.1]hept-5-en-2,3-dicarbonsäureimidophenyl)]methan, Bis[4-methallyl-methyl-bicyclo[2.2.1]hept-5-en-2,3-dicarbonsäureimidophenyl)]sulfon.

Als spezielle Beispiele für Maleinimide welche in den erfindungsgemässen Mischungen als Komponents (b) enthalten sein können, seinen genannt:
N,N'-Ethylen-bis-maleinimid,
N,N'-Hexamethylen-bis-maleinimid,
N,N'-m-Phenylen-bis-maleinimid,
N,N'-p-Phenylen-bis-maleinimid,
N,N'-4,4'-Diphenylmethan-bis-maleinimid,
N,N'-4,4'-3,3'-Dichlor-diphenylmethan-bis-maleinimid,
N,N'-4,4'-Diphenylether-bis-maleinimid,
N,N'-4,4'-Diphenylsulfon-bis-maleinimid,
N,N'-m-Xylylen-bis-maleinimid,
N,N'-p-Xylylen-bis-maleinimid,
N,N'-4,4'-2,2'-Diphenylpropan-bis-maleinimid,
das N,N'-Bis-maleinimid des 4,4'-Diamino-triphenylphosphats,
das N,N'-Bis-maleinimid des 4,4'-Diamino-triphenylphosphits,
das N,N'-Bis-maleinimid des 4,4'-Diamino-triphenylthiophosphats,
das N,N',N''-Trismaleinimid des Tris-(4-aminophenyl)-phosphats,
das N,N',N''-Trismaleinimid des Tris-(4-aminophenyl)-phosphits und
das N,N',N''-Trismaleinimid des Tris-(4-aminophenyl)-thiophosphats.

Bevorzugt werden als Komponente (b) Imide der Formeln IV oder V eingesetzt

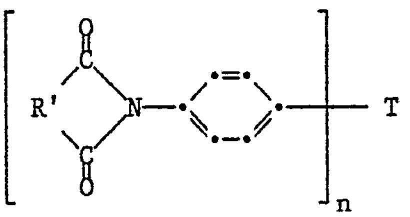 (IV), 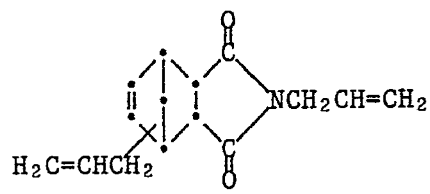 (V)

wobei R' für einen Rest

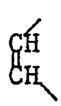 , 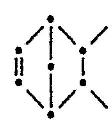

oder

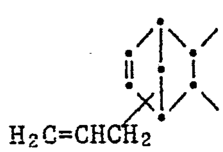

steht,
n 2 oder 3 bedeutet und T, bei n = 2;

-$CH_2$-, -O- oder 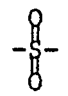

und, bei n = 3,

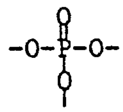

darstellt.

Besonders bevorzugte Komponenten (b) sind Bis[4-(allylbicyclo[2.2.1]hept-5-en-2,3-dicarbonsäureimidophenyl)]methan und N,N'-4,4'-Diphenylmethan-bis-maleinimid.

Als Komponente (c) können an sich beliebige bekannte Verbindungen eingesetzt werden, die mit den ungesättigten Imiden (b) und/oder den Imiden der Formel I zu reagieren vermögen. Besonders geeignete Komponenten (c) sind Diamine und Diole der Formel VI

$HQ-R^3-QH$ (VI),

worin Q O oder NH und $R_3$ einen zweiwertigen organischen Rest mit 2-30 C-Atomen bedeuten, sowie Phenol-und Kresol-Novolake oder Gemische solcher Verbindungen.

Bevorzugt stellt $R^3$ in Formel VI $-C_nH_{2n}$-mit m = 2-20, besonders $-(CH_2)_n$-mit n = 2-12, Arylen mit 6-10 C-Atomen, besonders m-oder p-Phenylen, Xylylen, Cyclopentylen, Cyclohexylen, 1,4-Bis(methylen)-cyclohexylen, den Rest des Bicyclohexylmethans oder einen Rest der Formel VII

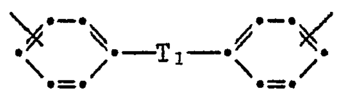 (VII)

mit $T_1$ = Methylen, Isopropyliden, CO, O, S oder $SO_2$ dar. Besonders bevorzugt ist $R^3$ ein in 4,4'-Stellung gebundener Rest der Formel VII.

$R^3$ kann eine geradkettige oder verzweigte Alkylengruppe mit 1-12 C-Atomen bedeuten, wie Methylen, Ethylen, Propylen, Butylen, Pentylen, Hexylen, 2-Ethyl-hexylen, Decylen und Dodecylen, vorzugsweise Alkylen mit 1-8 C-Atomen.

Eine weitere Klasse bevorzugter Komponenten (c) sind alkenylsubstituierte Phenole und Polyole. Als Beispiele seien genannt:

Verbindungen der Formel VIII

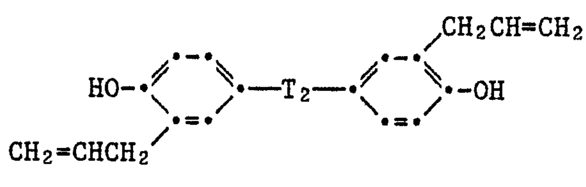 (VIII),

worin $T_2$ die direkte Bindung, Methylen, Isopropyliden, O, S, SO oder $SO_2$ bedeutet. Beispiele solcher Verbindungen sind: o,o'-Diallyl-Bisphenol A, Bis(4-hydroxy-3-allyl)biphenyl, Bis(4-hydroxy-3-allylphenyl)-methan und 2,2-Bis(4-hydroxy-3-allylphenyl)propan.

Propenylsubstituierte Phenole der Formel IX

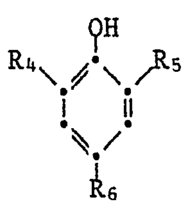 (IX),

worin $R_4$, $R_5$ und $R_6$ unabhängig voneinander ein Wasserstoffatom, eine Allyl-oder Propenylgruppe bedeuten, wobei mindestens eines von $R_4$ bis $R_6$ für die Propenylgruppe steht.

Verbindungen der Formel X

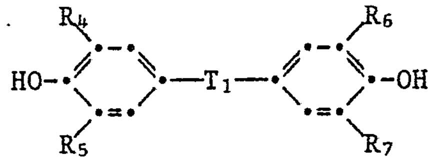 (X),

worin $R_4$, $R_5$, $R_6$ und $R_7$ unabhängig voneinander ein Wasserstoffatom, eine Allyl-oder Propenylgruppe bedeuten, wobei mindestens eines von $R_4$ bis $R_7$ für die Propenylgruppe steht und $T_1$ die oben unter Formel VII angegebene Bedeutung hat.
Verbindungen der Formel XI

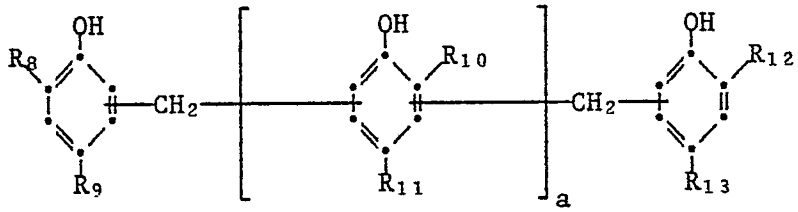 (XI),

worin $R_8$, $R_9$, $R_{10}$, $R_{11}$, $R_{12}$ und $R_{13}$ unabhängig voneinander ein Wasserstoffatom, Alkyl mit 1-4 C-Atomen, Allyl oder Propenyl bedeuten, wobei mindestens eines von $R_8$ bis $R_{13}$ für die Propenylgruppe steht und a einen Wert von 0 bis 10 darstellt. Bevorzugt sind Verbindungen der Formel X, worin $R_4$ und $R_6$ je eine Propenylgruppe und $R_5$ und $R_7$ je ein Wasserstoffatom bedeuten und $T_1$ für Methylen, Isopropyliden oder 0 steht.

Es können auch Isomerengemische aus propenyl-und allyl-substituierten ein-oder mehrwertigen Phenolen eingesetzt werden. Von den Isomerengemischen werden bevorzugt Mischungen aus propenyl-und allylsubstituierten Phenolen der Formel X eingesetzt, insbesondere solche, die durch partielle Isomerisierung von allylsubstituierten Phenolen der Formel Xa

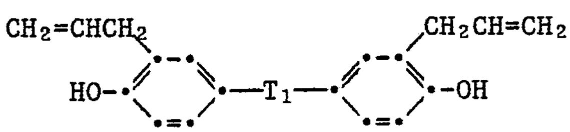 (Xa),

worin $T_1$ für Methylen, Isopropyliden oder 0 steht, erhalten werden. Derartige alkenylsubstituierte Phenole und Polyole sind z.B. in den Us-PS 4,100,140 und 4,371,6 = 719 beschrieben.

Als Komponenten (c) können ferner verwendet werden:
Azomethine der Formel XII oder XIII

$$R_{14}-\underset{R_{15}}{C}=N-R_{16} \quad (XII) \quad \text{oder} \quad R_{14}-\underset{R_{15}}{C}=N-R^3-N=\underset{R_{15}}{C}-R_{14} \quad (XIII),$$

worin $R_{14}$ ein Wasserstoffatom oder einen einwertigen Kohenwasserstoffrest darstellt und $R_{15}$ und $R_{16}$ die gleiche Bedeutung wie $R_{14}$ haben können, aber ungleich Wasserstoff sind oder $R_{14}$ und $R_{15}$ zusamment mit dem Bindungs-C-Atom ein cycloaliphatisches Ringsystem bilden und $R^3$ die unter Formel VI angegebene Bedeutung hat. Derartige Verbindungen sind z.B. in der GB-PS 1 443 067 beschrieben.

Bevorzugt sind Verbindungen der Formel XII und besonders XIII, worin $R_{14}$ Wasserstoff, $R_{15}$ und $R_{16}$ Phenyl und $R^3$ $-(CH_2)_m-$ mit m = 2-8, m-oder p-Phenylen oder einen Rest der Formel VII darstellen. Als Beispiele solcher Azomethine seien genannt:
1,6-Benzyliden-hexamethylenediamin,
N,N'-Benzyliden-4,4'-diaminodiphenylmethan,
N,N'-Benzyliden-p-phenylendiamin und Benzalanilin.
Dihydrazide der Formel XIV

$$H_2N-NH-\overset{O}{\overset{\|}{C}}-D-\overset{O}{\overset{\|}{C}}-NH-NH_2 \quad (XIV),$$

worin D die direkte Bindung oder einen zweiwertigen organischen Rest, vor allem den Rest einer aliphatischen, cycloaliphatischen oder aromatischen Dicarbonsäure bedeutet. Als Beispiele von Verbindungen der Formel XIV seien genannt: Oxalsäure-, Malonsäure-, Bernsteinsäure-, Glutarsäure-, Adipinsäure-, Pimelinsäure-, Korksäure, Sebacinsäure-, Cyclohexandicarbonsäure-, Terephthalsäure-, Isophthalsäure-, 2,6-und 2,7-Naphthalindicarbonsäuredihydrazid und Gemische solcher Dihydrazide.

Hydrazide von Aminosäuren der Formel XV

$H_2N\text{-}D_1\text{-}\overset{O}{\overset{\|}{C}}\text{-}NH\text{-}NH_2$ (XV),

worin $D_1$ einen zweiwertigen organischen Rest bedeutet, wie die Hydrazide der folgenden Aminosäuren: Aminoessigsäure, Alanin, Leucin, Isoleucin, Phenylalanin, Valin, $\beta$-Alanin, $\gamma$-Aminobuttersäure, $\alpha$-Aminobuttersäure, Epsilon-Aminocapronsäure, Aminovaleriansäure, m-Aminobenzoesäure, Anthranilsäure oder Hydrazide von Aminosäuren der Formel XVI

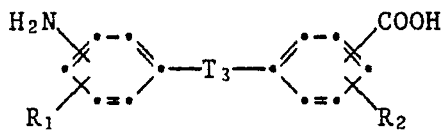

(XVI),

worin $T_3$ die direkte Bindung, O, S, $SO_2$ oder $CH_2$ und $R_1$ und $R_2$ unabhängig voneinander ein Wasserstoffatom oder eine Methylgruppe bedeuten.

Schliesslich können als Komponenten c) auch Aminocrotonsäuerederivate der in den US-PS 4,089,845 und 4,247,672 beschriebenen Art verwendet werden.

Bevorzugt enthalten die erfindungsgemässen Stoffgemische als Komponente (c) Phenol-oder Kresol-Novolake, Verbindungen der Formel VIa

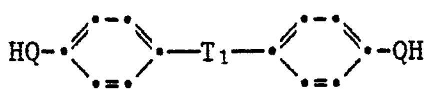

(VIa),

worin Q und $T_1$ die oben unter der Formel VI bzw. VII engegebene Bedeutung haben und $T_1$ insbesondere Methylen oder Isopropyliden darstellt, oder Verbindungen der Formel VIII, worin $T_2$ Isopropyliden ist, sowie Gemische der genannten bevorzugten Verbindungen. Besonders bevorzugt als Komponente (c) ist 4,4'-Diaminodiphenylmethan. Die Verbindung der Formeln VI bis XVI sind bekannt oder können nach an sich bekannten Methoden hergestellt werden.

Die Komponenten (a) werden vorzugsweise in einer Menge von 20-50 Gew.% und die Komponenten (b) in einer Menge von 50-80 Gew.% eingesetzt. Der Anteil an Komponenten (c) beträgt mit Vorteil 0-25 Gew.% und insbesondere 0-15 Gew.%, bezogen auf die Summe von (a) + (b).

Die Herstellung der erfindungsgemässen Stoffgemische kann auf an sich bekannte Weise durch Vermahlen und Vermengen oder durch Aufschmelzen der Komponenten vorgenommen werden. Den erfindungsgemässen Gemischen können auch übliche Zusatzstoffe, wie Füllstoffe, Weichmacher, Pigmente, Farbstoffe, Formtrennmittel und flammhemmende Stoffe, zugesetzt werden.

Die Härtung bzw. Verarbeitung der Stoffgemische kann in einem inerten organischen Lösungsmittel, vorzugsweise jedoch aus der Schmelze und gegebenenfalls in Gegenwart eines Härtungskatalysators erfolgen. Als inerte organische Lösungsmittel eignen sich z.B. Dimethylformamid, Dimethylacetamid, N-Methylpyrrolidon, Toluol, Xylole, Methylethylketon, und Ethylenglykolmonoalkyl-oder -dialkylether mit 1-4 C-Atomen in den Alkylgruppen. Je nach Art der eingesetzten Komponente (c) und der beabsichtigten Verwendung kommen als Härtungskatalysatoren z.B. organische Peroxide, wie Di-tert-butyl peroxid, Dicumylperoxid oder tert-Butylperbenzoat, oder basische Katalysatoren, vor allem primäre, sekundäre und tertiäre Amine in Betracht, wie z.B. Diethylamin, Tributylamin, Triethylamin, Benzylamin, N,N,N',N'-Tetramethyl-4,4'-diaminodiphenylmethan, N,N-Diisobutylaminoacetonitril, N,N-Dibutylaminoacetonitril und heterocyclische Basen, wie Chinolin, N-Methylpyrrolidon und Imidazol. Besonders bei der Verwendung von Phenolen oder Polyolen als Komponente (c) empfiehlt sich der Zusatz von basischen Katalysatoren der erwähnten Art. Die Härtung wird im allgemeinen bei Temperaturen zwischen 150 und 350°C, besonders 180 und 300°C vorgenommen.

Die erfindungsgemässen Stoffgemische können ferner leicht aus der Schmelze, besonders auch ohne Zusatz von schwerflüchtigen Lösungsmitteln, appliziert werden, beispielsweise zum Imprägnieren von Glasfaser-, Kohlenstoffaser-oder Aramidfaser-Geweben, wie Fasergewebe aus den unter dem Handelsnamen Kevlar® bekannten Poly(1,4-phenylenterephthalamiden).

Die erfindungsgemässen Stoffgemische können vielseitig angewendet werden, z.B. als Laminier-oder Elektroharze, als Hochtemperatur-Klebstoffe oder zur Herstellung von Beschichtungen oder Formkörpern, wie Prepregs und Verbundwerkstoffen. Gegenstand der Erfindung sind somit auch die durch Härtung der erfindungsgemässen Gemische erhaltenen Formkörper, Beschichtungen oder Verklebungen.

Die Erfindung wird durch die folgenden Beispiele näher erläutert.

## Herstellungsbeispiele

### Beispiel 1

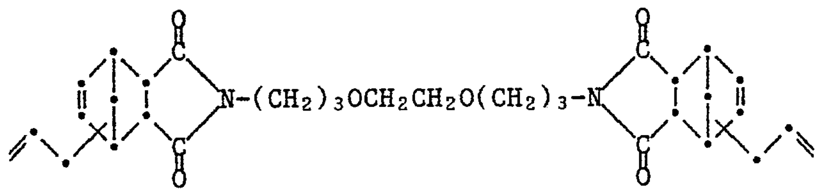

In einem mit absteigendem Kühler, Rühler, Tropftrichter, Gaseinleitungsrohr und Thermometer versehenen Rührkolben legt man 306 g Allyl-bicyclo[2.2.1]hept-5-en-2,3-dicarbonsäureanhydrid (Allylnadicanhydrid, hergestellt gemäss US-PS 3,105,839) vor, gibt 0,219 g der Verbindung der Formel

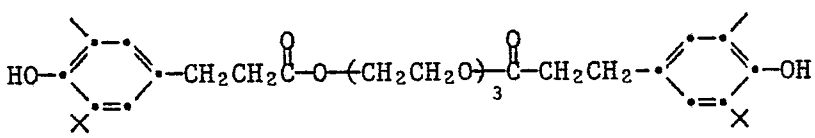
(Antioxidans Nr.1)

zu und erhitzt unter Rühren und Durchleiten von Stickstoff auf 120°C. Danach tropft man 132 g 4,7-Dioxadecan-1,10-diamin der Firma BASF im Lauf von 65 Minuten ein. Die Reaktion ist leicht exotherm. Man steigert die Temperatur auf 200°C, wobei 25 g Wasser abdestillieren, erniedrigt den Druck auf 4 Pa und hält diese Bedingungen während 5 Stunden 15 Minuten aufrecht. Man erhält ein sehr zähes, hellbraunes Harz mit einer Viskosität von 1,5 Pa.s bei 80°C in quantitativer Ausbeute.

| Elementaranalyse: | ber. für $C_{32}H_{40}N_2O_6$ | gefunden: |
|---|---|---|
| % C: | 70,05 | 69,87 |
| % H: | 7,35 | 7,44 |
| % N: | 5,11 | 5,04 |

Infrarotspektrum: 1130 $cm^{-1}$ Ethergruppen
1180 $cm^{-1}$ Ethergruppen
1640 $cm^{-1}$ Allylgruppe
1698 $cm^{-1}$ Carbonylgruppen
1767 $cm^{-1}$ Carbonylgruppen
2890 $cm^{-1}$ CH-Schwingung
2944 $cm^{-1}$ CH-Schwingung

Beispiel 2:

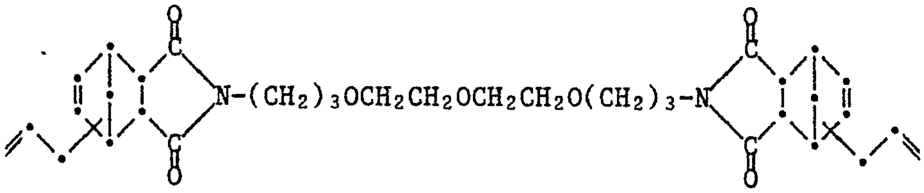

Man setzt 306 g Allylnadic-anhydrid mit 165 g, 4,7,10-Trioxatridecan-1,13-diamin der Firma BASF so um, wie dies im Beispiel 1 beschrieben ist. Man führt die Imidisierung während 2 Stunden 50 Minuten bei 210°C und 2,6 Pa zu Ende. Man erhält 418,5 g (94,3 % d.Th.) eines braunen, sehr zähen Harzes mit einer Viskosität von 1,53 Pa.s bei 80°C.

| Elementaranalyse: | ber. für $C_{34}H_{44}N_2O_7$ | gefunden: |
|---|---|---|
| % C: | 68,90 | 68,32 |
| % H: | 7,48 | 7,45 |
| % N: | 4,73 | 4,82 |

Beispiel 3:

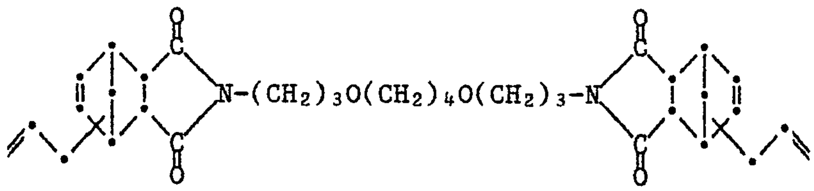

Man setzt 102 g Allylnadic-anhydrid mit 51 g 4,9-Dioxadodecan-1,12-diamin der Firma BASF so um, wie dies im Beispiel 1 beschrieben ist. Nachkondensation während 1 Stunde 50 Minuten bei 200°C und 5 Pa. Man erhält 141,2 g (98 % d.Th.) eines hellbraunen Harzes mit einer Viskosität von 0,40 Pa.s bei 80°C.

| Elementaranalyse: | ber. für $C_{34}H_{44}O_6N_2$ | gefunden: |
|---|---|---|
| % C: | 70,81 | 70,82 |
| % H: | 7,69 | 7,70 |
| % N: | 4,86 | 4,81 |
| $\bar{M}_n$ | 576,4 | 628 |
| $\bar{M}_w$ | --- | 904 |

Beispiel 4:

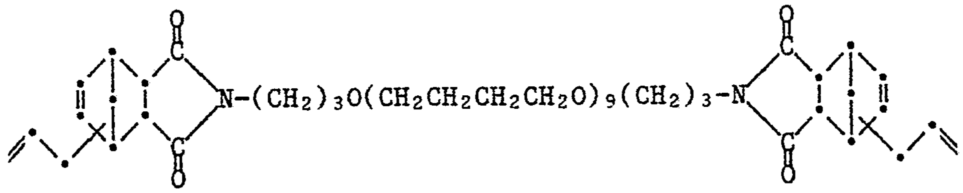

Man setzt 563,9 g Bis(3-aminopropyl)polytetrahydrofuran 750 der Firma BASF mit 306 g Allylnadicanhydrid in Gegenwart von 0,435 g Antioxidans 1 so um, wie dies im Beispiel 1 beschrieben ist. Nachkondensation während 3 Stunden 45 Minuten bei 200°C und 5 Pa. Man erhält 830,5 g (98,5 % d.Th.) eines zähflüssigen Harzes mit folgenden Kenndaten:

$\eta_{80}$ = 265 mPa•s
$\bar{M}_n$ = 1335
$\bar{M}_w$ = 2964.

<u>Anwendungsbeispiele</u>

| Beispiel Nr. (härtbares Stoffgemisch) | bestehend aus: |
| --- | --- |
| A | 40 g Produkt gem. Beispiel 1<br>60 g Imid I[1)] |
| B | 30 g Produkt gem. Beispiel 2<br>70 g Imid I[1)] |
| C | 50 g Produkt gem. Beispiel 3<br>50 g Imid I[1)] |
| D | 50 g Produkt gem. Beispiel 4<br>50 g Imid I[1)] |
| E | 80 g Produkt gem. Beispiel 3<br>20 g Imid II[2)] |
| F | 67 g Produkt gem. Beispiel 2<br>33 g Imid III[3)] |
| G | 67 g Produkt gem. Beispiel 3<br>33 g Imid III[3)] |
| H | 70 g Produkt gem. Beispiel 1<br>23,4 g Imid III[3)]<br>6,6 g DDM[4)] |
| I | 80 g Produkt gem. Beispiel 2<br>15,6 g Imid III[3)]<br>4,4 g DDM[4)] |

Erläuterung:

1) Imid I =

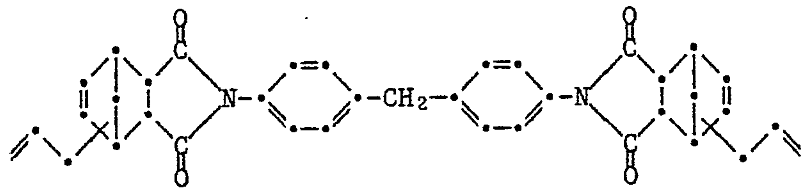

2) Imid II =

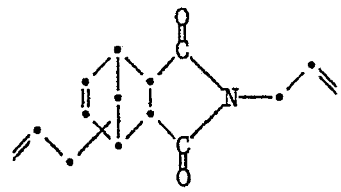

3) Imid III =

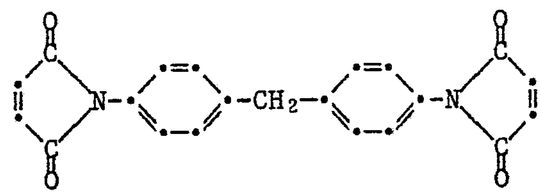

4) D.D.M. =

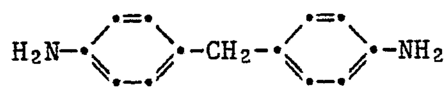

Verfahren:

Die Mischungen A-I werden bei etwa 150°C zusammengeschmolzen. Die rotbraunen, homogenen Mischungen werden im Vakuum entgast, in Stahlformen von 120 x 120 x 4 $mm^3$ gegossen und 3 Stunden bei 200°, 3 Stunden bei 200° und 12 Stunden bei 250°C gehärtet. Man erhält einwandfreie Platten, die zu Norm-Prüfstäben zerschnitten werden.

Eigenschaften von Festkörpern aus den Mischungen A-I

| Anwendungs-beispiel | $T_G$ 5) (°C) | Schlagbiege-festigkeit 6) ($kJ/m^2$) | Biege-festigkeit 7) ($N/mm^2$) | Randfaser-dehnung 7) (%) |
|---|---|---|---|---|
| A | 308 | 17,6 | 114,4 | 5,24 |
| B | 311 | 18,6 | 121,4 | 4,33 |
| C | 260 | 21,4 | 121,3 | 6,57 |
| D | 230 | 16,6 | 79,0 | 6,25 |
| E | 213 | 22,5 | 91,2 | 5,23 |
| F | >250 | 17,6 | 115,2 | 7,20 |
| G | >250 | 26,9 | 117,1 | 7,53 |
| H | 232 | 25,4 | 127,6 | 6,91 |
| I | 173 | 38,2 | 127,6 | 7,50 |

5) TA 3000 (TMA) Mettler AG, CH
6) DIN 53453
7) DIN 53452

**Ansprüche**

1. Imide der Formel I

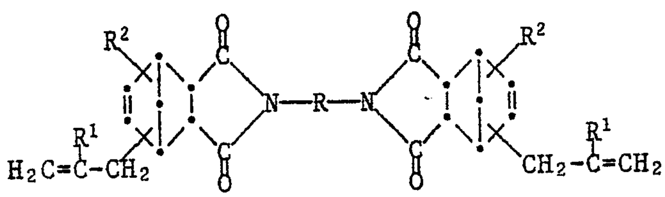

(I),

worin $R^1$ und $R^2$ unabhängig voneinander ein Wasserstoffatom oder Methyl bedeuten, R einen (Poly)oxa-alkylenrest der Formel

$$-[(C_pH_{2p}O)_s-(C_qH_{2q}O)_t-C_rH_{2r}]-$$

darstellt mit p, q und r = unabhängig voneinander eine ganze Zahl von 2 bis 6, s = 0 oder 1 und t = 1-30.

2. Imide der Formel I nach Anspruch 1, worin $R^1$ und $R^2$ ein Wasserstoffatom bedeuten.

3. Imide der Formel I nach Anspruch 1, worin s = 1, die Indizes p und r gleich sind und t = 1-20.

4. Imide der Formel I nach Anspruch 3, worin p und r jeweils 2 oder insbesondere 3 sind, q 2 bis 4 ist und t = 1-10.

5. Imide der Formel I nach Anspruch 1, worin s = 0, die Indizes q und r gleich sind und t = 1-20.

6. Imide der Formel I nach Anspruch 5, worin q und r jeweils 2, 3 oder 4 sind und t = 1-10.

7. Imide der Formel I nach Anspruch 4, worin R $-(CH_2)_3OCH_2CH_2O(CH_2)_3-$, $-(CH_2)_3OCH_2CH_2CH_2CH_2O-(CH_2)_3-$, $-(CH_2)_3O(CH_2CH_2O)_2(CH_2)_3-$oder $-(CH_2)_3O(CH_2CH_2CH_2CH_2O)_9(CH_2)_3-$ist.

8. Imide der Formel I nach Anspruch 6, worin R $-(CH_2CH_2O)_tCH_2CH_2-$, $-(CH_2CH_2CH_2O)_tCH_2CH_2CH_2-$,

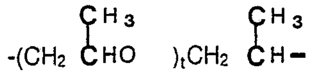

oder $-(CH_2CH_2CH_2CH_2O)_tCH_2CH_2CH_2CH_2-$ist.

9. Verfahren zur Herstellung von Imiden der Formel I nach Anspruch 1, dadurch gekennzeichnet, dass man eine Anhydrid der Formel II

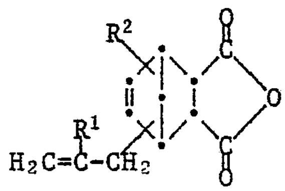

(II)

bei erhöhter Temperatur und unter Abdestillieren des bei der Reaktion entstehenden Wassers mit einem Diamin der Formel III

$H_2N-R-NH_2$ (III)

umsetzt, wobei für $R^1$, $R^2$ und R das im Anspruch 1 Angegebene gilt.

10. Polymere, die dadurch erhältlich sind, dass man mindestens ein Imid der Formel I nach Anspruch 1 auf Temperaturen zwischen 180 und 300°C erhitzt.

11. Heisshärtbares Stoffgemisch enthaltend

(a) 10-90 Gew.% mindestens eines Imids der Formel I nach Anspruch 1,

(b) 90-10 Gew.% mindestens eines ungesättigten Imids verschieden von (a) und, bezogen auf die Summe von (a) und (b),

(c) 0-30 Gew.% mindestens einer zur Reaktion mit der Komponente (a) und/oder (b) befähigten Verbindung.

12. Stoffgemisch nach Anspruch 11, worin die Komponente (b) eine Verbindung der Formeln IV oder V ist

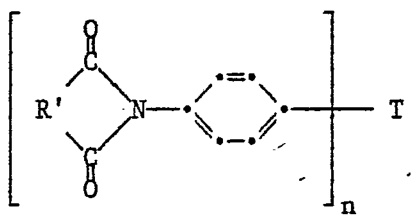

(IV),

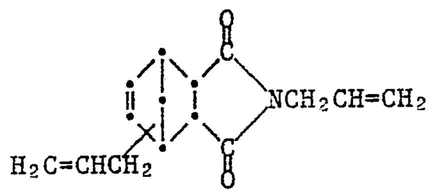

(V)

wobei R' für einen Rest

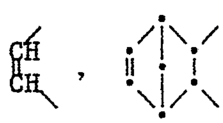

oder

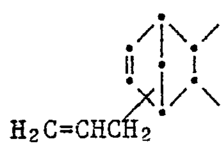

steht,

n 2 oder 3 bedeutet und T, bei n = 2,

$-CH_2-$, $-O-$ oder 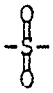

und, bei n = 3,

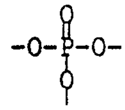

darstellt.

13. Stoffgemisch nach Anspruch 11, worin die Komponente (c) ein Diamin oder ein Diol der Formel VI ist

$HQ-R^3-QH$ (VI),

worin Q O oder NH und $R^3$ einen zweiwertigen organischen Rest mit 2 bis 30 C-Atomen bedeuten, sowie Phenol-und Kresol-Novolake oder Gemische solcher Verbindungen.

14. Die aus den härtbaren Stoffgemischen nach Anspruch 11 durch Härtung erhaltenen Formförper, Beschichtungen oder Verklebungen.